# EUROPEAN PATENT SPECIFICATION

(11) **EP 2 848 608 B1**
(45) Date of publication and mention of the grant of the patent: **20.12.2017**
(21) Application number: 14174623.0
(22) Date of filing: 26.06.2014
(51) Int. Cl.: C07D 213/30, A61K 31/4409, A61K 31/555, A61P 31/04, A61P 31/10

(54) **SILVER COMPLEX COMPOUNDS, METHOD FOR THEIR PRODUCTION AND THEIR USE**
Silberkomplexverbindungen, Verfahren zu deren Herstellung und deren Verwendung
Composés complexes d'argent, procédé pour leur production et leur utilisation

(30) Priority: 08.07.2013 PL 40458313
(43) Date of publication of application: 18.03.2015
(73) Proprietor: Uniwersytet Medyczny W Lodzi, 90-419 Lodz (PL)
(72) Inventor: Ochocki, Justyn, Lodz (PL); Kalinowska-Lis, Urszula, Lodz (PL)
(74) Representative: Kaminski, Piotr

(56) References cited:
- WO-A1-2008/145759
- KULIG J ET AL: "Potentiometric studies on complexes of silver(I) in solutions. Part III. Reactions of Ag(I) complexing with some pyridine derivatives in aqueous solutions", POLISH JOURNAL OF CHEMISTRY, vol. 59, no. 10-12, 1986, pages 1029-1037, XP009182444, ISSN: 0137-5083
- M. S. SUN ET AL: "Comparative formation constants for complexes of copper, nickel, and silver with some substituted pyridines", CANADIAN JOURNAL OF CHEMISTRY, vol. 45, no. 22, 15 November 1967 (1967-11-15), pages 2729-2739, XP055167995, ISSN: 0008-4042, DOI: 10.1139/v67-443
- URSZULA KALINOWSKA-LIS ET AL: "Synthesis, characterization and antimicrobial activity of silver(I) complexes of hydroxymethyl derivatives of pyridine and benzimidazole", JOURNAL OF ORGANOMETALLIC CHEMISTRY, vol. 749, 1 January 2014 (2014-01-01), pages 394-399, XP055167851, ISSN: 0022-328X, DOI: 10.1016/j.jorganchem.2013.10.035
- T. R. DEBOER ET AL: "Supramolecular Assembly of Ag(I) Centers: Diverse Topologies Directed by Anionic Interactions", CRYSTAL GROWTH & DESIGN, vol. 14, no. 10, 11 September 2014 (2014-09-11), pages 4901-4905, XP055168041, ISSN: 1528-7483, DOI: 10.1021/cg501175g & T R Deboer ET AL: "Electronic Supplementary Information Supramolecular Assembly of Ag(I) Centers: Diverse Topologies Directed by Anionic Interactions", , 11 September 2014 (2014-09-11), XP055168051, Retrieved from the Internet: URL:http://pubs.acs.org/doi/suppl/10.1021/ cg501175g/suppl_file/cg501175g_si_001.pdf [retrieved on 2015-02-06]

## Description

The invention relates to silver complex, method of silver compound preparation and use of silver compound.

Silver(I) complexes of pyridine and use thereof in preparation of pharmaceutical compositions for treatment or prevention of infections are well known. US 2010/0234339 application discloses silver(I) complexes with pyridine derivatives substituted with carboxyl groups, especially silver(I) complexes with nicotinic and isonicotinic acid derivatives.

Silver(I) complexes with 4-(hydroxymethyl)pyridine have been known from the work of Kulig J et al. ("Potentiometric studies on complexes of silver (I) in solutions, Part III, Reactions of Ag(I) complexing with some pyridine derivatives in aqueous solutions (Polish Journal of Chemistry, vol. 59, no. 10-12, 1986) and M.S. Sun et al. ("Comparative formation constants for complexes of copper, nickel and silver with some substituted pyridines", Canadian Journal of Chemistry, vol. 45, no 22, November 15, 1967). The pharmaceutical use of said silver complexes has not been disclosed. WO 2008/145759 discloses use of pyridine silver complexes in treatment or prevention of infections, however complexes disclosed do not contain 4-hydroxymethyl group.

The aim of this invention is to provide new use of silver complex and method of silver compound preparation.

Silver complex for use according to present invention is characterized in that, it is a silver complex of 4-(hydroxymethyl)pyridine. Preferably, it contains silver and 4-(hydroxymethyl)pyridine in 1:2 molar ratio. In particular, it contains silver on +1 formal oxidation state coordinated by 4-(hydroxymethyl)pyridine, and contains counterion, wherein said counterion is an acid anion selected from group consisting of hardly oxidizable non-organic acids, tetrafluoroboric acid, carboxylic acids, alkylsulfonic acids, arylsulfonic acids. The counterion is in particular an anion of an acid selected from group consisting of non-organic oxoacids, where central atom of an element surrounded by oxygen atoms is on +5, +6 or +7 oxidation state, tetrafluoroboric acid, C1-6 alkylmonocarboxylic acids, C1-6 alkyldicarboxylic acids, C1-6 alkyltricarboxylic acids, C1-6 alkylsulfonic acids, C6-11 arylsulfonic acids.

Preferably, silver coordination compound is a compound of formula 1:

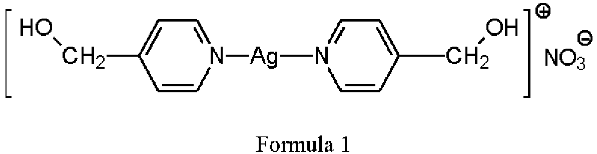

Method of silver complex of 4-(hydroxymethyl)pyridine preparation for use according to present invention is characterized in that, 4-(hydroxymethyl)pyridine and silver(I) compound at least partially soluble in reaction environment are used as a reagents, which reagents are combined together in reaction environment containing at least one non-amine polar solvent in temperature 0-50°C, reaction is conducted for 1-180 minutes while keeping 0-50°C temperature during mechanical stimulation of reaction environment, and subsequently silver complex of 4-(hydroxymethyl)pyridine is obtained. Preferably, non-amine polar solvent selected from group consisting of inorganic, non-amine hydroxyl solvents and organic non-amine hydroxyl solvents is used as a non-amine polar solvent. In particular, solvent selected from a group consisting of water, lower aliphatic alcohols and mixtures thereof is used as a non-amine hydroxyl solvent.

Preferably, silver compound and 4-(hydroxymethyl)pyridine are used in 1:2 molar ratio. In particular, reaction is conducted for 15-120 minutes while keeping temperature on 20-30°C. Silver complex is isolated by evaporation of solvent/solvents to dryness.

Preferably, silver(I) nitrate(V) is used as a silver(I) compound. In particular, purified water is used as a solvent, and silver complex isolated after water evaporation is crystallized from ethanol.

Preferably, isolated silver complex containing negatively charged counterion is subjected to ion exchange with substitution of previously inserted counterion as a counterion of initial silver(I) compound by different counterion selected from group consisting of anions of hardly oxidizable non-organic acids, tetrafluoroboric acid, carboxylic acids, alkylsulfonic acids and arylsulfonic acids.

The compound according to present invention is used for preparation of formulation for inhibition of pathogenic cells growth, for prevention or treatment of animal or human organism. Preferably, it is used for inhibition of bacterial cells development. Optionally, it is used for inhibition of fungal cells development.

Preferably, silver complex containing silver and 4-(hydroxymethyl)pyridine is used in 1:2 molar ratio. In particular, used is a compound containing silver on +1 formal oxidation state coordinated by 4-(hydroxymethyl)pyridine, and containing counterion, which counter anion is an acid anion selected from a group consisting of hardly oxidizable non-organic acids, tetrafluoroboric acid, carboxylic acids, alkylsulfonic acids, arylsulfonic acids. Used is in particular silver complex compound where counterion is an acid anion selected from a group consisting of non-organic oxoacids, wherein central atom of element surrounded by oxygen atoms is on +5, +6 or +7 oxidation state, tetrafluoroboric acid, C1-6 alkylmonocarboxylic acids; C1-6 alkyldicarboxylic acids, C1-6 alkyltricarboxylic acids, C1-6 alkylsulfonic acids, C6-11 arylsulfonic acids.

Preferably, silver complex of formula 1 is used:

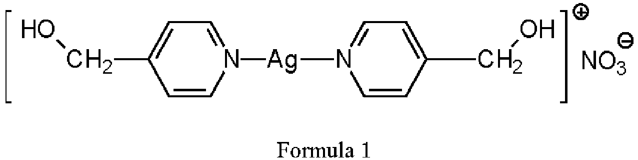

Silver complex is prepared using method according to the invention in preferred embodiment in an one-step process, most preferably in water, thus without a need of use of toxic organic solvent. Moreover process is highly efficient, fast and proceeds in mild conditions - in ambient temperature - thus does not require complex equipment nor handling.

Silver complex according to invention has also preferable physicochemical properties, for example presents good solubility in water (and also in organic solvents), which facilitates use of compound according to invention as a supplement for medical preparations or medical materials.

The compound is especially predestined to such use, while it exhibits antibacterial and antifungal activity characteristic for silver(I) compounds. Thus, it can be applied as an active compound in preparations and materials intended for use, inter alia in dermatology, dentistry, laryngology, ophthalmology and surgery. Moreover, compound according to the invention can be applied as an antibacterial and/or antifungal agent added to ointments, gels, liquids etc., intended for external use, and as a component added to bandage materials.

Silver complex according to invention is prepared in reaction of silver(I) compound with 4-(hydroxymethyl)pyridine by combining said reagents in reaction environment containing at least one non-amine polar solvent. The "term non-amine polar solvent" as used in description of the invention and claims covers polar solvent which does not contain nitro-substituent, wherein nitrogen atom contains free electron pair. This is because in presence of such groups reaction of silver(I) compound with 4-(hydroxymethyl)pyridine can be impaired. Moreover, from the scope covered by this term, solvents containing groups with highly polarized heteroatom-oxygen bond (as, for example, phosphoric acid hexamethyltriamide) are excluded, because in presence of such groups reaction of silver(I) with 4-(hydroxymethyl)pyridine can also be impaired. Preferably, non-amine polar solvent is a solvent chosen from a group comprising inorganic hydroxyl solvents and organic non-amine hydroxyl solvents, and also mixtures thereof, and especially solvent chosen from a group consisting of water, lower aliphatic alcohols and any mixture of water and lower aliphatic alcohols. Particularly preferable solvent is purified water, like demineralised water or distilled water.

Silver(I) compound used in a method according to present invention is silver(I) compound at least partially soluble in reaction environment containing at least one non-amine polar solvent. Preferably, silver(I) compound is silver(I) nitrate(V) or silver(I) acetate, and more preferably silver(I) nitrate(V).

Reagents, i.e. silver compound and 4-(hydroxymethyl)pyridine are combined in reaction environment in substantially any ratio, but to obtain high efficiency (in relation to load level for both reagents) and to provide high purity of final silver complex, silver(I) compound and 4-(hydroxymethyl)pyridine is preferably combined in molar proportion possibly close to 1:2. Hereby, molar proportion should be understood as a proportion of Ag+ ion moles to amount of 4-(hydroxymethyl)pyridine moles.

Reaction is conducted in 0-50°C temperature for 1-180 minutes with mechanical stimulation of environment, for example through mixing. Preferably, reaction is conducted for 15-120 minutes in 20-30°C temperature, especially in ambient temperature. Subsequently silver complex compound is isolated by, for example, evaporation of solvent to dryness.

In method according to invention silver complex is obtained, in which coordination cation, containing silver(I) atom coordinated by 4-(hydroxymethyl)pyridine, is accompanied by counterion - anion previously inserted to the reaction environment together with reagent - initial silver(I) salt. The invention provides for the possibility of silver(I) complex transformation into a next silver(I) complex according to the invention, wherein counterion is an anion of an acid selected from the group consisting of hardly oxidizable non-organic acids, tetrafluoroboric acid, carboxylic acids, alkylsulfonic acids and arylsulfonic acids, where said anion is different than anion introduced previously to reaction environment together with initial silver(I) salt. Preferably, the counterion is an anion of acid selected from group consisting of inorganic oxoacids, where central atom of element surrounded by oxygen atoms is on +5, +6 or +7 oxidation state, tetrafluoroboric acid, C1-6 alkylmonocarboxylic acids, C1-6 alkyldicarboxylic acids, C1-6 alkyltricarboxylic acids, C1-6 alkylsulfonic acids, C6-11 arylsulfonic acids.

As an example of acid selected form the group comprising non-organic oxoacids, where central atom of the element surrounded by oxygen atom is on +5, +6 or +7 oxidation state, chloric(V), chloric(VII), sulfuric(VI) and optionally nitric(V) acid can be indicated (in case where initial silver(I) compound is a compound different then silver(I) nitrate(V).

As an example of an acid selected from the group comprising C1-6 alkylmonocarboxylic acids, lactic acid and acetic acid can be indicated (in case where initial silver(I) compound is a different compound than silver(I) acetate).

As an example of an acid selected from the group comprising C1-6 alkyldicarboxylic acids, succinic acid and tartaric acid can be indicated.

As an example of an acid selected from the group comprising C1-6 alkyltricarboxylic acids, citric acid can be indicated.

As an example of acid selected from the group comprising C1-6 alkylsulfonic acids methanesulfonic acid can be indicated.

As an example of acid selected from the group comprising C6-11 alkylsulfonic benzenesulfonic acid, toluenesulfonic acid, naphtalenesulfonic acid, methylnaphtalenesulfonic acid can be indicated.

For this purpose, within present invention, conduction of ion exchange of silver complex compound anion according to the present invention is foreseen using ion-exchange carriers or using ion pairs extraction method.

Therefore, according to the invention, silver complex of 4-(hydroxymethyl)pyridine is provided, wherein said compound preferably contains cation, which consists of silver atom on +1 formal oxidation state coordinated by 4-(hydroxymethyl)pyridine, and contains counterion, which is an anion of acid selected from group consisting of hardly oxidizable non-organic acids, tetrafluoroboric acid, carboxylic acids, alkylsulfonic acids and arylsulfonic acids. Preferably, the counterion is an anion of acid selected from a group consisting of inorganic oxoacids, where central atom of an element surrounded by oxygen atoms is on +5, +6 or +7 oxidation state (such as chloric(V), chloric(VII), sulfuric(VI), (V)), tetrafluoroboric acid, C1-6 alkylmonocarboxylic acids (such as acetic and lactic acid), C1-6 alkyldicarboxylic acids (such as succinic, tataric), C1-6 alkyltricarboxylic acids (such as citric acid), C1-6 alkylsulfonic acid (such as methanesulfonic acid, ethanesulfonic acid), C6-11 arylsulfonic acid (such as benzenesulfonic acid, toluenesulfonic acid, naphtalenesulfonic acid, methylnaphtalenesulfonic acid.

Particularly preferably, silver coordination compound is a compound of formula 1:

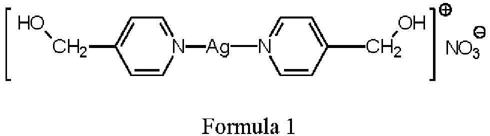

### Example

### The method of silver complex compound preparation

Solution comprising 4-(hydroxymethyl)pyridine (2 mmol, 0.218 g) in 10 cm3 of distilled water is placed on magnetic stirrer. One portion of silver(I) nitrate(V) solution (1 mmol, 0.170 g) in 5 cm3 of distilled water is added while mixing. Reaction mixture is protected from light using aluminium foil cover and reaction is performed for 1 hour with mixing in ambient temperature (24°C). Water is evaporated till dryness on rotary evaporator under reduced pressure at 60°C temperature. Obtained white precipitate is further crystallized from hot ethanol. After around 1 day colourless crystals are separated, flushed with anhydrous ethyl ether and dried in air (83% efficiency).

Properties: colourless crystals, soluble in H2O, ethanol, acetone, dimethyl sulfoxide.
1H NMR (300 MHz, D2O): δ (ppm) = 4,73 (s, 4H, 2 x OCH2); 7,49 (d, 4H, 2 x (py)H(3)H(5), 3JHH= 4,76 Hz); 8.49 (d, 4H, 2 x (py)H(2)H(6), 3JHH = 4,76 Hz).
13C NMR (600 MHz, D2O): δ (ppm) = 61,94 (CH2OH); 122,49 (C(3, 5)); 151,13 (C(4)); 153,17 (C(2, 6)).
IR (KBr) Vmax (cm-1): (O-H, C-H) 3140 (s, br), 2909 (m), 2824 (m), (C=C, C=N) 1611 (s), 1561 (m); (NO3) 1378 (vs), (C-O) 1059 (m), 1004 (m); (vs: very strong; s: strong; m: medium; w: weak; vw: very weak; br: broad).
MS-electrospray: ESI+-MS (H2O) m/z: 326 [Ag(4-CH2OHpy)2]+, 217 [Ag(4-CH2OHpy)]+.
Elemental analysis; calculated for C12H14N3O5Ag: C 37,13; H 3,64%; N 10,83%; determined: C 37,14%, H 3,43%; N 10,87%.

## Claims

1. Silver complex of 4-(hydroxymethyl)pyridine for use in inhibition of pathogenic cells growth in prevention or treatment of animal or human organism.

2. Compound for use according to claim 1, wherein said compound contains silver and 4-(hydroxymethyl)pyridine in 1:2 molar ratio.

3. Compound for use according to claim 2, wherein said compound contains silver of +1 formal oxidation state coordinated by 4-(hydroxymethyl)pyridine, and contains counterion, which is an anion of acid selected from group consisting of hardly oxidizable inorganic acids, tetrafluoroboric acid, carboxylic acids, alkylsulfonic acids, arylsulfonic acids.

4. Compound for use according to claim 3, wherein said counterion is an anion of acid selected from group consisting of inorganic oxoacids, wherein central atom of element surrounded by oxygen atoms is on +5, +6 or +7 oxidation state, tetrafluoroboric acid, C1-6 alkylmonocarboxylic acids, C1-6 alkyldicarboxylic acids, C1-6 alkyltricarboxylic acids, C1-6 alkylsulfonic acids, C6-11 arylsulfonic acids.

5. Compound for use according to any one of claims 1 to 4, wherein said compound is represented by formula 1:

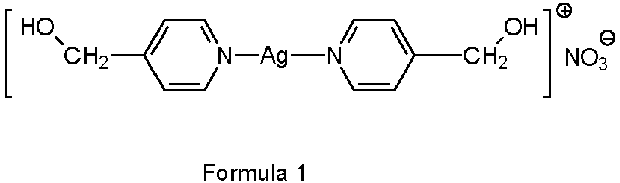

6. Compound for use according to any one of claims 1 to 5, wherein said cells are bacterial cells or fungal cells.

7. Preparation method of silver complex of 4-(hydroxymethyl)pyridine according to claim 1 **characterized in that** 4-(hydroxymethyl)pyridine and silver(I) compound at least partially soluble in reaction environment are used as reagents, which reagents are combined together in reaction environment containing at least one non-amine polar solvent in temperature 0-50°C, reaction is conducted for 1-180 minutes while keeping the temperature at 0-50°C, with mechanical stimulation of reaction environment, and subsequently silver complex of 4-(hydroxymethyl)pyridine is isolated.

8. Method according to claim 7 **characterized in that** as said non-amine polar solvent used is a non-amine hydroxyl solvent selected from a group consisting of inorganic non-amine hydroxyl solvents, and organic non-amine hydroxyl solvents is used as a non-amine polar solvent, preferably said non-amine hydroxyl solvent is selected from a group consisting of water, lower aliphatic alcohols and mixtures thereof.

9. Method according to any one of either claim 7 or 8 **characterized in that** silver(I) compound and 4-(hydroxymethyl)pyridine are used in 1:2 molar ratio.

10. Method according to any one of claims 7 to 9 **characterized in that** reaction is conducted for 15-120 minutes while keeping 20-30°C temperature.

11. Method according to any one of claims 7 to 10 **characterized in that** silver complex is isolated by evaporation of solvent/solvents to dryness.

12. Method according to any one of claims 7 to 11 **characterized in that** silver(I) nitrate(V) is used as a silver(I) compound.

13. Method according to claim 12 **characterized in that** purified water is used as a solvent and silver complex compound isolated after water evaporation is crystallized from ethanol.

14. Method according to any one of claims 7-13 **characterized in that** isolated silver complex compound containing negatively charged counterion is subjected to ion exchange with substitution of previously inserted counterion as a counterion of initial silver(I) compound by a different counterion selected from group containing anions of hardly oxidizable inorganic acids, tetrafluoroboric acid, carboxylic acids, alkylsulfonic acids and arylsulfonic acids.

## Patentansprüche

1. Silberkomplex von 4- (Hydroxymethyl)pyridin zur Verwendung bei der Hemmung von Wachstum der pathogenen Zellen bei der Prävention oder Behandlung von tierischen oder menschlichen Organismus.

2. Verbindung zur Verwendung nach Anspruch 1, wobei die Verbindung Silber und 4-(Hydroxymethyl)pyridin in einem molaren Verhältnis von 1:2 enthält.

3. Verbindung zur Verwendung nach Anspruch 2, wobei die vorgenannte Verbindung Silber eines formalen Oxidationsstufe +1 koordiniert mit 4-(hydroxymethyl)pyridin enthält und ein Gegenion, das ein Anion der Säure aus der Gruppe bestehend aus schwer oxidierbaren anorganischen Säuren, Tetrafluorborsäure, Carbonsäuren, Alkylsulfonsäuren, Arylsulfonsäuren ausgewählten enthält.

4. Verbindung zur Verwendung nach Anspruch 3, wobei das Gegenion ein Anion der Säure aus der Gruppe bestehend aus anorganischen Oxosäuren, wobei das Zentralatom des Elements mit Sauerstoffatomen auf +5, +6 oder +7 Oxidationsstufe umgeben ist, Tetrafluorborsäure, C1 -6 Alkylmonocarbonsäuren, C1-6 Alkyldicarbonsäuren, C1-6 Alkyltricarboxylicsäuren, C1-6 Alkylsulfonsäuren, C6-11 Arylsulfonsäuren ausgewählten ist.

5. Verbindung zur Verwendung nach einem der Ansprüche 1 bis 4, wobei die vorgenannte Verbindung durch Formel Idargestellt wird:

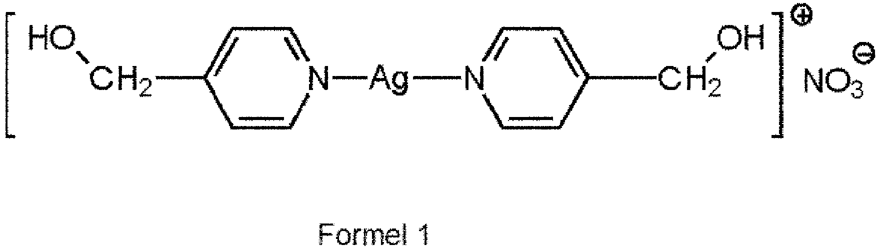

6. Verbindung zur Verwendung nach einem der Ansprüche 1 bis 5, wobei die vorgenannte Zellen die Bakterienzellen oder Pilzzellen sind.

7. Herstellungsverfahren für Silberkomplex von 4- (Hydroxymethyl)pyridine nach Anspruch 1, **dadurch gekennzeichnet, dass** 4- (Hydroxymethyl)pyridin und Silber (I) -Verbindung, zumindest teilweise löslich in Reaktionsumgebung, als Reagenzien verwendet werden, wobei die Reagenzien zusammen in Reaktionsumgebung, mindestens ein polares nicht-Amin Lösungsmittel enthaltend, in Temperatur von 0-50°C kombiniert werden, die Reaktion für 1-180 Minuten durchgeführt wird, während die Temperatur bei 0-50°C gehalten wird, mit mechanischer Stimulation des Reaktionumgebung und anschließend Silberkomplex von 4-(Hydroxymethyl)pyridin isoliert wird.

8. Verfahren nach Anspruch 7, **dadurch gekennzeichnet, dass** das verwendete vorgenannte nicht-Amin polare Lösungsmittel ein nicht-Amin-Hydroxyl Lösungsmittel aus einer Gruppe bestehend aus anorganischen nicht-Amin-Hydroxyl-Lösungsmittel und organischen nicht-Amin-Hydroxyl-Lösungsmittel ausgewählt ist, wird als nicht-Amin polaren Lösungsmittel verwendet, wobei vorzugsweise das vorgenannte nicht-Amin-Hydroxyl Lösungsmittel aus der Gruppe bestehend aus Wasser, niederen aliphatischen Alkoholen und Mischungen davon ausgewählt ist.

9. Verfahren nach einem der beiden Ansprüche 7 oder 8, **dadurch gekennzeichnet, dass** Silber (I)-Verbindung und 4-(Hydroxymethyl)pyridin in einem 1:2 molaren Verhältnis verwendet werden.

10. Verfahren nach einem der Ansprüche 7 bis 9, **dadurch gekennzeichnet, dass** die Reaktion für 15-120 Minuten durchgeführt wird, während die Temperatur von 20-30°C gehalten wird.

11. Verfahren nach einem der Ansprüche 7 bis 10, **dadurch gekennzeichnet, dass** Silberkomplex durch Verdampfen des Lösungsmittels/der Lösungsmittel bis zur Trockne isoliert wird.

12. Verfahren nach einem der Ansprüche 7 bis 11, **dadurch gekennzeichnet, dass** Silber (I) nitrat (V) als Silber (I)-Verbindung verwendet wird.

13. Verfahren nach Anspruch 12, **dadurch gekennzeichnet, dass** gereinigtes Wasser als Lösungsmittel verwendet wird und Silberkomplexverbindung isolierte nach Verdampfen von Wasser aus Ethanol kristallisiert wird.

14. Verfahren nach einem der Ansprüche 7-13, **dadurch gekennzeichnet, dass** isolierte Silberkomplexverbindung negativ geladenen Gegenion enthaltend, einem Ionenaustausch mit Substitution des zuvor eingebauten Gegenion als ein Gegenion der anfänglichen Silber (I) Verbindung zu einem anderen Gegenion aus der Gruppe bestehend aus Anionen der kaum oxidierbarer anorganischen Säuren, Tetrafluorborsäure; Carbonsäuren, Alkylsulfonsäuren und Arylsulfonsäuren.ausgewählten unterworfen wird.

## Revendications

1. Complexe d'argent de la 4- (hydroxyméthyl) pyridine destiné à être utilisé dans l'inhibition de la croissance de cellules pathogènes dans la prévention ou le traitement d'un organisme animal ou humain.

2. Composé destiné à être utilisé selon la revendication 1, où ledit composé contient de l'argent et de la 4- (hydroxyméthyl) pyridine en un rapport molaire de 1 : 2.

3. Composé destiné à être utilisé selon la revendication 2, où ledit composé contient de l'argent d'un état d'oxydation formel +1 coordonné par la 4- (hydroxyméthyl) pyridine, et contient un contre-ion, qui est un anion d'acide choisi dans le groupe constitué par les acides inorganiques difficilement oxydables, l'acide tétrafluoroborique, les acides carboxyliques, les acides alkylsulfoniques, les acides arylsulfoniques.

4. Composé destiné à être utilisé selon la revendication 3, où ledit contre-ion est un anion d'acide choisi parmi le groupe constitué par les oxacides inorganiques, où l'atome central de l'élément entouré par des atomes d'oxygène est sur l'état d'oxydation +5, +6 ou +7, l'acide tétrafluoroborique, les acides alkylcarboxyliques en C1-6, les acides alkyldicarboxyliques en C1-6, les acides alkyltricarboxyliques en C1-6, les acides alkylsulfoniques en C1-6, les acides arylsulfoniques en C6-11.

5. Composé destiné à être utilisé selon l'une quelconque des revendications de 1 à 4, où ledit composé est représenté par la formule 1:

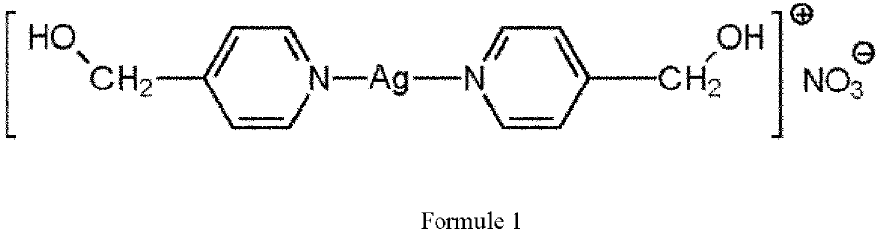

6. Composé destiné à être utilisé selon l'une quelconque des revendications de 1 à 5, où lesdites cellules sont des cellules bactériennes ou les cellules fongiques.

7. Procédé de préparation d'un complexe d'argent de 4- (hydroxyméthyl) pyridine selon la revendication 1, **caractérisé en ce que** la 4- (hydroxyméthyl) pyridine et le composé d'argent (I) au moins partiellement solubles dans l'environnement réactionnel sont utilisés comme réactifs, lesquels réactifs sont combinés ensemble dans un environnement réactionnel contenant au moins un solvant polaire non-amine à la température de 0-50°C, la réaction est conduite pendant 1-180 minutes tout en maintenant la température à 0-50°C, avec stimulation mécanique de l'environnement réactionnel, et par la suite le complexe d'argent de la 4-(hydroxyméthyl) pyridine est isolé.

8. Procédé selon la revendication 7, **caractérisé en ce que** comme ledit solvant polaire non-aminé utilisé est un solvant hydroxyle non-aminé choisi dans le groupe constitué par les solvants hydroxyles non-aminés inorganiques et les solvants hydroxyles non-aminés organiques est utilisé comme solvant polaire non-aminé, de préférence ledit solvant hydroxyle non-aminé est choisi dans le groupe constitué par l'eau, des alcools aliphatiques inférieurs et leurs mélanges.

9. Procédé selon l'une quelconque des revendications 7 ou 8, **caractérisé en ce que** le composé d'argent (1) et la 4- (hydroxyméthyl) pyridine sont utilisés en un rapport molaire de 1 : 2.

10. Procédé selon l'une quelconque des revendications de 7 à 9, **caractérisé en ce que** la réaction est conduite pendant 15 à 120 minutes en maintenant la température à 20-30°C.

11. Procédé selon l'une quelconque des revendications de 7 à 10, **caractérisé en ce que** le complexe d'argent est isolé par évaporation du solvant / solvants jusqu'à siccité.

12. Procédé selon l'une quelconque des revendications de 7 à 11, **caractérisé en ce que** le nitrate (V) d'argent (I) est utilisé comme composé d'argent (I).

13. Procédé selon la revendication 12, **caractérisé en ce que** l'eau purifiée est utilisée comme solvant et le composé complexe d'argent isolé après évaporation de l'eau est cristallisé à partir d'éthanol.

14. Procédé selon l'une quelconque des revendications de 7 à 13, **caractérisé en ce que** le composé complexe d'argent isolé contenant un contre-ion chargé négativement est soumis à un échange d'ions avec substitution du contre-ion précédemment inséré comme contre-ion du composé d'argent (I) initial par un contre-ion différent choisi dans le groupe constitué par des anions d'acides inorganiques difficilement oxydables, d'acide tétrafluoroborique, d'acides carboxyliques, d'acides alkylsulfoniques et d'acides arylsulfoniques.
